# EUROPEAN PATENT SPECIFICATION

(11) **EP 2 698 422 B1**
(45) Date of publication and mention of the grant of the patent: **22.06.2016**
(21) Application number: 12770825.3
(22) Date of filing: 02.04.2012
(51) Int. Cl.: C12M 1/00, A61K 35/14, A61M 1/34, A61M 1/36, C12N 5/00, G01N 33/49

(54) **MONONUCLEAR CELL PREPARATION MATERIAL AND MONONUCLEAR CELL PREPARATION METHOD USING SAME**
MATERIAL ZUR HERSTELLUNG MONONUKLEÄRER ZELLEN UND VERFAHREN ZUR HERSTELLUNG MONONUKLEÄRER ZELLEN DARAUS
PRÉPARATION DE MATÉRIAU DE CELLULES MONONUCLÉAIRES ET PROCÉDÉ DE PRÉPARATION DE CELLULES MONONUCLÉAIRES UTILISANT CELLE-CI

(30) Priority: 11.04.2011 JP 2011087789
(43) Date of publication of application: 19.02.2014
(73) Proprietor: Kaneka Corporation, Osaka-shi, Osaka 530-8288 (JP)
(72) Inventor: UMEDA, Nobuyoshi, Settsu-shi Osaka 566-0072 (JP); SATO, Nobuhiko, Settsu-shi Osaka 566-0072 (JP); TSUKAMOTO, Ayako, Settsu-shi Osaka 566-0072 (JP)
(74) Representative: Hoffmann Eitle
(86) International application number: PCT/JP2012/058977
(87) International publication number: WO 2012/141032

(56) References cited:
- EP-A1- 2 450 431
- WO-A1-2006/025371
- WO-A1-2006/093205
- WO-A1-2012/070622
- JP-A- 2 193 069
- JP-A- 9 075 076
- JP-A- 2004 121 144
- JP-A- 2007 289 076
- JP-A- 2008 022 822
- JP-A- 2008 043 895
- JP-A- 2008 086 235
- JP-B2- 3 938 973
- US-A1- 2002 045 091
- HIROSHI MIYAMOTO ET AL.: 'Preliminary studies for the development of a second generation granulocytapheresis (G-CAP) column' JOURNAL OF ARTIFICIAL ORGANS vol. 13, 2010, pages 92 - 96, XP019806542
- ANTON BRUIL ET AL.: 'The Mechanisms of Leukocyte Removal by Filtration' TRANSFUSION MEDICINE REVIEWS vol. IX, no. 2, 1995, pages 145 - 166, XP005441359

## Description

### TECHNICAL FIELD

The present invention relates to use of a mononuclear cell preparation material capable of easy and efficient preparation of mononuclear cells in a method for preparing mononuclear cells. More specifically, the present invention relates to use of a mononuclear cell preparation material capable of reducing granulocyte contamination in a method for preparing mononuclear cells.

### BACKGROUND ART

In recent years, transplantation of mononuclear cell fractions of bone marrow, umbilical cord blood, and peripheral blood has been used in clinical practice for ischemic diseases such as cerebral infarction, myocardial infarction, and limb ischemia. It is believed that stem cells including mesenchymal stem cells, hematopoietic stem cells, and endothelial progenitor cells in such mononuclear cell fractions promote angiogenesis or nerve regeneration, thereby producing a therapeutic effect.

For example, Taguchi et al. demonstrates that transplantation of a bone marrow mononuclear cell fraction containing CD34 positive cells to patients with a peripheral arterial occlusive disease, Buerger's disease results in increased angiogenesis and therefore can be an effective way to treat Buerger's disease (Non Patent Literature 1). Their other study reports that transplantation of mononuclear cells from bone marrow is an effective treatment for patients with acute ischemic diseases such as cerebral infarction or ischemic heart diseases (Non Patent Literature 2).

In order to prepare mononuclear cells, granulocytes, which can cause side effects such as inflammation and reduce the therapeutic effect of mononuclear cells, should be removed. Currently, the removal of granulocytes is accomplished by centrifugation or density-gradient centrifugation using an isolation medium. The density-gradient centrifugation method, however, has some problems, for example: it requires time-consuming processes such as time-consuming centrifugation and complicated operations, has high inter-operator variability, and needs operations in an open system, and the use of this method for cell therapy requires a large facility called cell processing center (CPC).

A method for removing granulocytes without using centrifugation proposed so far is to use a nonwoven fabric made of a material, such as polyester, which selectively adsorbs granulocytes (Patent Literature 1). This removal method easily removes granulocytes in a closed system without the need of special equipment, and therefore can be used in general medical facilities such as municipal hospitals. Additionally, the processing time of this method is shorter than that of the density-gradient centrifugation method. However, unfortunately, since nonwoven fabrics have high porosity, mononuclear cells also remain in the nonwoven fabric, resulting in a reduced recovery of mononuclear cells.

Moreover, a known strategy to recover necessary cells remaining in a nonwoven fabric is to wash the nonwoven fabric with a physiological solution containing dextran (Patent Literature 2). However, since recovery solutions containing dextran are highly viscous, the viability of cells may be reduced.

Additionally, it is known that divalent cations such as calcium ion and magnesium ion activate granulocytes, and the activated granulocytes more strongly adhere to nonwoven fabrics (Non Patent Literature 3). So far, there is no study in which a physiological solution containing a divalent cation is used to wash a nonwoven fabric to increase the mononuclear cell recovery while reducing granulocyte contamination.

### CITATION LIST

### PATENT LITERATURE

Patent Literature 1: WO 06/093205
Patent Literature 2: JP 3938973 B

### NON PATENT LITERATURE

Non Patent Literature 1: A. Taguchi et al. , Eur. J. Vasc. Endovasc. Surg. 2003, 25, 276-278
Non Patent Literature 2: A. Taguchi et al., Stem Cells. 2010, 28, 1292-1302
Non Patent Literature 3: A. Bruil et al., Transfusion Med. Rev. 1995, 9, 145-166

### SUMMARY OF INVENTION

### TECHNICAL PROBLEM

The present inventors have made various studies to provide a mononuclear cell preparation material capable of efficiently preparing mononuclear cells and a method for preparing mononuclear cells using such a material. In particular, a strategy designed and examined by the present inventors to increase the mononuclear cell recovery while reducing granulocyte contamination is to pass blood through a nonwoven fabric and subsequently flow a washing solution that has no negative effect on cells, to further recover mononuclear cells remaining in voids of the nonwoven fabric. However, it was revealed that not only mononuclear cells but also granulocytes are washed out by flowing the washing solution according to this strategy. Further, a strategy designed and examined by the present inventors to improve the adhesion of granulocytes to a nonwoven fabric to prevent granulocytes from being washed out is to pass blood containing a divalent cation through the nonwoven fabric. However, this strategy was also found to allow granulocytes to be washed out.

Accordingly, an object of the present invention is to provide a mononuclear cell preparation material capable of increasing the mononuclear cell recovery while preventing granulocyte contamination, and a mononuclear cell preparation method which increases the mononuclear cell recovery.

### SOLUTION TO PROBLEM

The present inventors have intensively studied to provide a mononuclear cell preparation material capable of efficiently preparing mononuclear cells while reducing granulocyte contamination, which has been difficult to achieve, and a method for preparing mononuclear cells using such a material. Finally, the present inventors have found that the use of a nonwoven fabric having specific properties enables efficient preparation of mononuclear cells while reducing granulocyte contamination. Additionally, it has also been found that the addition of a divalent cation such as calcium ion or magnesium ion in a washing solution, not in blood, enables efficient preparation of mononuclear cells while reducing granulocyte contamination. Thus, the present invention has been completed.

Specifically, the present specification describes a mononuclear cell preparation material for removing granulocytes from blood to prepare mononuclear cells, the material including a nonwoven fabric made of a polyamide resin and having an air permeability of at least 10 mL/cm²/sec but not more than 75 mL/cm²/sec.

The present specification also describes a mononuclear cell preparation device, including a container provided with an inlet and an outlet and packed with the mononuclear cell preparation material.

The present invention relates to a mononuclear cell preparation method as defined in the claims, including the steps of:
(A) passing blood through the mononuclear cell preparation device from an inlet side to an outlet side of the device, thereby capturing granulocytes in the mononuclear cell preparation device; and
(B) passing a washing solution through the mononuclear cell preparation device from the inlet side to the outlet side of the device, thereby recovering mononuclear cells remaining in the mononuclear cell preparation device.

Preferably, the method further includes, before the step (A), the step of priming.

The washing solution contains a divalent cation.

The divalent cation is preferably calcium ion or magnesium ion.

Preferably, the divalent cation is present at a concentration of not more than 500 mEq/L.

It is preferred that a mononuclear cell preparation flow-through from the outlet side of the mononuclear cell preparation device have a ratio of a mononuclear cell recovery to a granulocyte recovery of not less than 3.5.

### ADVANTAGEOUS EFFECTS OF INVENTION

The present invention increases the mononuclear cell recovery while reducing granulocyte contamination, and therefore enables more efficient preparation of mononuclear cell fractions. Additionally, the present invention enables efficient preparation of a necessary amount of mononuclear cell fraction from a small amount of blood, and therefore is expected to reduce burdens on donors.

### BRIEF DESCRIPTION OF DRAWINGS

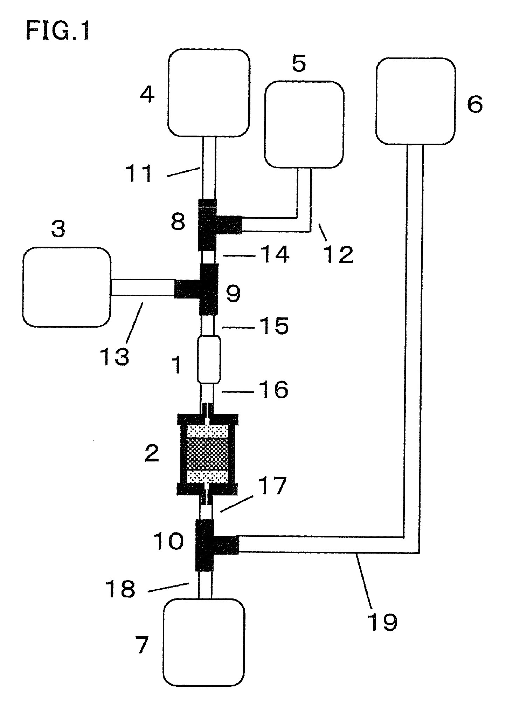

Fig. 1 shows an example of mononuclear cell preparation lines.

### DESCRIPTION OF EMBODIMENTS

The following description is offered to illustrate the present invention in more detail. It should be noted that the present invention is not limited only to specific examples shown below.

The present specification describes a mononuclear cell preparation material for removing granulocytes from blood to collect mononuclear cells, which includes a nonwoven fabric made of a polyamide resin.

The term "blood" herein is not limited to peripheral blood, bone marrow, umbilical cord blood, and menstrual blood, and is intended to also include amniotic fluid, tissue extracts, and partially purified products, processed products, and cultured products of these. The animal origin of blood is not limited at all, and examples thereof include mammals such as humans, bovines, mice, rats, swine, monkeys, dogs, and cats. Since the mononuclear cell preparation material may be used to provide mononuclear cells which are to be returned to mammals mainly for the treatment of ischemic diseases or the like, that is, to be used as medicaments for ischemic diseases, blood rich in stem cells (e.g. hematopoietic stem cells and mesenchymal stem cells), i.e. bone marrow, umbilical cord blood, or amniotic fluid, is preferably used.

Optionally, various anticoagulants and the like may be added to blood. Examples of compounds usable in blood include heparin, low-molecular-weight heparin, Futhan (nafamostat mesilate), EDTA, ACD (acid-citrate-dextrose) solution, CPD (citrate-phosphate-dextrose) solution, and CPDA (citrate-phosphate-dextrose-adenine) solution.

In the present invention, for the purpose of the treatment of diseases such as ischemic diseases, it is preferred that blood be used within 48 hours of collection. However, even when refrigerated or frozen blood is thawed and then treated, a similar level of granulocyte removal efficiency can be achieved. Thus, blood stored in any condition can be used.

Specific examples of mononuclear cells that can be prepared using the mononuclear cell preparation material include monocytes and lymphocytes. Specific examples of granulocytes that can be removed by the mononuclear cell preparation material include neutrophils, eosinophils, basophils, and immature cells of these.

The term "nonwoven fabric" herein refers to a fabric prepared by chemically, thermally, or mechanically combining fibers or fiber aggregates without weaving or knitting. Fibers maintaining a certain shape due to, for example, the friction between mutually contacting fibers or mutual entanglement of fibers are also included as mechanically combined fibers.

The mononuclear cell preparation material includes a nonwoven fabric made of a polyamide resin. Especially, it is preferred that the main component of the nonwoven fabric be a polyamide resin (i.e. the amount thereof is not less than 50% by weight). Examples of polyamide resins include nylon and aramid, and those of any type can be used without limitation. Preferred are nylon resins such as nylon 6, nylon 66, nylon 11, nylon 12, and nylon 46, which are easily formed into nonwoven fabrics. In particular, nylon 6 is particularly preferred for reasons of granulocyte removal efficiency. These materials may be used alone, or alternatively some of these materials may be combined, mixed, or fused before use. Additionally, molecules having affinity for certain cells, such as proteins, peptides, amino acids, or saccharides, may be immobilized on fibers of the nonwoven fabric as necessary. The use of such a polyamide resin prevents granulocytes from being washed out with an applied washing solution and thereby contaminating a flow-through from a mononuclear cell preparation device, and therefore can enable high-yield, efficient preparation of mononuclear cells while reducing granulocyte contamination.

Nonwoven fabrics having an air permeability of at least 10 mL/cm²/sec but not more than 75 mL/cm²/sec can be used. The air permeability is preferably at least 15 mL/cm²/sec but not more than 70 mL/cm²/sec, and more preferably at least 20 mL/cm²/sec but not more than 65 mL/cm²/sec. A nonwoven fabric having an air permeability of less than 10 mL/cm²/sec may not allow mononuclear cells to pass through it because of its too small apertures, so that a flow-through from a mononuclear cell preparation device tends to have a reduced recovery of mononuclear cells. Conversely, a nonwoven fabric having an air permeability of more than 75 mL/cm²/sec may allow part of granulocytes in applied blood to pass through a mononuclear cell preparation device without contacting the nonwoven fabric because of its too large apertures, and therefore tends to increase the granulocyte recovery.

The term "air permeability" herein is a value obtained by measurement with a Frazier air permeability tester according to the method described below. First, a sample is held between clamp jaws of a tester, and an induced draft fan is controlled such that an inclined manometer reads 125 Pa. Next, the amount of air passing through the sample is determined from the pressure read by a vertical manometer at that time and the kind of the used orifice with reference to a table attached to the tester.

The fiber diameter of the nonwoven fabric is not limited at all, and is preferably not more than 25 µm. For reasons of granulocyte removal efficiency, the fiber diameter is more preferably not more than 20 µm, and still more preferably not more than 16 µm. The fiber diameter refers to the width of fibers perpendicular to the fiber axis. The fiber diameter can be determined by photographing a separation material including a nonwoven fabric with a scanning electron microscope; calculating the diameters of fibers based on a magnification scale of the photograph; and averaging the calculated diameters. The fiber diameter is preferably the average of 50 or more fibers, and more preferably the average of 100 or more fibers. It should be noted that when, for example, some fibers are overlapped, or the measurement of the width of a fiber is impeded by other fibers and thus is impossible, or a fiber that remarkably differs in diameter is present together, the data of such fibers are not used to calculate the fiber diameter. Also, in the case where the nonwoven fabric includes fibers of multiple types which largely differ in width, for example, differ in diameter by more than 7 µm, the fiber diameter is calculated for each fiber type and then the smaller fiber diameter is regarded as the fiber diameter of the nonwoven fabric because fibers with a smaller diameter have stronger influence on the granulocyte capturing efficiency.

For practical reasons, the mononuclear cell preparation material is packed in a container provided with an inlet and an outlet for blood before use. The mononuclear cell preparation material may be used either in the form of a flat cut sheet of an appropriate size or in a rolled-up form to treat blood.

When the mononuclear cell preparation material is packed into a container provided with an inlet and an outlet, the mononuclear cell preparation material may or may not be compressed. In a preferred example of the usage of the mononuclear cell preparation material, the mononuclear cell preparation material made of a nonwoven fabric is preferably cut into pieces of an appropriate size, and then used as a single layer or a laminate of layers with a thickness of about 1-200 mm. For reasons of granulocyte capturing efficiency, the thickness is more preferably 2 mm to 150 mm, and further more preferably 3 mm to 100 mm. The granulocyte preparation material may be rolled up and then packed into a container. In the case where the mononuclear cell preparation material is used in a rolled-up form, blood may be treated by passing the blood through this roll from the inside to the outside or conversely from the outside to the inside to separate blood cells.

The shape, size, and material of the container to be packed with the mononuclear cell preparation material are not particularly limited. The shape of the container may be any shape such as spherical, container-shaped, cassette-shaped, bag-shaped, tubular, or columnar. Specific preferred examples of the container include, but are not limited to, a translucent tubular container having a diameter of about 10 mm to about 50 mm and a height of about 1 mm to about 50 mm; and an about 1-50 mm-thick quadratic prism-shaped container having rectangular or square faces with sides having a length of about 10 mm to about 50 mm.

The container may be made of any structural material. Specific examples of structural materials that can form the container include nonreactive polymers, biocompatible metals and alloys, and glasses. Examples of nonreactive polymers include acrylonitrile polymers (e.g. acrylonitrile butadiene styrene terpolymer), halogenated polymers (e.g. polytetrafluoroethylene, polychlorotrifluoroethylene, tetrafluoroethylene-hexafluoropropylene copolymer, polyvinyl chloride), polyamide, polyimide, polysulfone, polycarbonate, polyethylene, polypropylene, polyvinyl chloride-acrylic copolymer, polycarbonate acrylonitrile butadiene styrene, polystyrene, and polymethylpentene. Examples of useful metal materials (biocompatible metals and alloys) for the container include stainless steel, titanium, platinum, tantalum, gold, and alloys of these, gold plated ferroalloy, platinum plated ferroalloy, cobalt chromium alloy, and titanium nitride-coated stainless steel. Especially preferred are materials having sterilization resistance, such as polypropylene, polyvinyl chloride, polyethylene, polyimide, polycarbonate, polysulfone, and polymethylpentene.

The mononuclear cell preparation method of the present invention characteristically includes the steps of: (A) passing blood through the mononuclear cell preparation device from an inlet side to an outlet side of the device, thereby capturing granulocytes in the mononuclear cell preparation device; and (B) passing a washing solution through the mononuclear cell preparation device from the inlet side to the outlet side of the device, thereby recovering mononuclear cells remaining in the mononuclear cell preparation device. Blood and a washing solution may be passed through the device in a direction, for example, downward under gravity, upward against gravity, or horizontally. In order for blood to spread to the entire mononuclear cell separation material more uniformly, blood is preferably run downward under gravity or upward against gravity.

The flow rate of blood and the method of passing blood through the device are not limited at all. For example, blood may be passed through the device under gravity, or with a roller clamp or a syringe pump at a constant flow rate, or blood may all be passed through the device at once by applying high pressure. For reasons of granulocyte removal efficiency, it is preferable to pass blood through the device with a syringe pump at a constant flow rate. It is more preferable to pass blood through the device with a syringe pump at a flow rate of not more than 2.5 mL/min. This allows blood to slowly contact the nonwoven fabric and thus enables the nonwoven fabric to capture more granulocytes.

Preferably, the mononuclear cell preparation method includes the step of applying a priming solution to the mononuclear cell preparation device before applying blood, to remove the air in the mononuclear cell preparation device to improve the granulocyte removal efficiency and ensure paths for blood. The priming solution is not limited at all, and physiological saline and buffers are preferred. For reasons of simplicity and workability of the line system, the priming solution is more preferably the same solution as the washing solution. For practical reasons, the volume of the priming solution is preferably about 1 to 100 times the capacity of the container to be packed with the mononuclear cell preparation material.

For example, the washing solution may effectively be physiological saline, a liquid containing a divalent cation, a saccharide, serum, and/or a protein, a buffer, a medium, plasma, or a liquid containing these. In particular, the use of a washing solution containing a divalent cation instead of adding a divalent cation to blood improves the adhesion of granulocytes to the nonwoven fabric, and therefore enables efficient preparation of mononuclear cells while reducing granulocyte contamination. Examples of such divalent cations include calcium ion, magnesium ion, manganese ion, zinc ion, nickel ion, and barium ion, which are preferred for reasons of granulocyte removal efficiency. Calcium ion and magnesium ion, which have been used for injection, are more preferred. The divalent cation concentration is preferably not more than 500 mEq/L. The concentration is more preferably not more than 360 mEq/L, and still more preferably not more than 180 mEq/L. If the concentration is higher than 500 mEq/L, the cation has non-negligible negative effects on cells. The unit, mEq/L, represents a multiplication of mmol/L by the ion valency.

Preferred washing solutions are solutions that have been used for medical purposes and have no negative effect on cells, and examples thereof include physiological saline containing calcium chloride and/or magnesium sulfate and Ringer solution.

According to the present invention, mononuclear cells remaining in the mononuclear cell separation device can be recovered by applying a washing solution after passing blood through the device. Particularly in the case where only a small amount of blood is treated, the ratio of blood remaining in the mononuclear cell separation device to the treated amount can be substantially high because of high porosity of general nonwoven fabrics. Accordingly, it is very important to recover mononuclear cells remaining in the mononuclear cell separation device by the washing step. Generally, however, when a washing solution is applied, granulocytes as well as mononuclear cells remaining in a mononuclear cell separation device can then be washed out. In contrast, the use of a polyamide resin according to the present invention makes it possible to, when applying a washing solution, recover mononuclear cells remaining in a mononuclear cell separation device without washing out granulocytes captured in the mononuclear cell preparation device.

The flow rate of the washing solution and the method of passing the washing solution through the device are not limited at all. For example, the washing solution may be passed through the device under gravity, or with a roller clamp or a syringe pump at a constant flow rate, or the washing solution may all be passed through the device at once by applying high pressure, as mentioned for passing blood through the device.

The amount of blood to be treated is preferably 1 mL to 100 mL, and for use in the treatment of diseases such as ischemic diseases, the amount is more preferably 2 mL to 80 mL, and still more preferably 3 mL to 60 mL. On the other hand, the amount of the washing solution to be used is preferably at least 1 but less than 20 times the capacity of the mononuclear cell preparation device. The amount is more preferably at least 2 but less than 15 times, and still more preferably at least 3 but less than 10 times the capacity of the mononuclear cell preparation device. If the amount is less than 1 time the capacity, blood remaining in the mononuclear cell preparation device is unlikely to be completely subjected to displacement. This tends to cause a flow-through from the mononuclear cell preparation device to have a reduced recovery of mononuclear cells. Conversely, if the amount is more than 20 times the capacity, the recovered blood tends to be too diluted.

In the case where a flow-through from the mononuclear cell preparation device obtained according to the present invention is to be returned to mammals mainly for the treatment of diseases such as ischemic diseases, that is, to be used as a medicament for ischemic diseases, a closed line system is preferably used to aseptically prepare mononuclear cells.

In the case of using a line system, various lines can be formed. Generally, a means for storing blood and a means for storing the washing solution are provided on the inlet side of the mononuclear cell preparation device. In order to perform operations aseptically, it is preferable to provide separate lines respectively connected to the means for storing blood and the means for storing the washing solution. Or, a single line may be used for both the means for storing blood and the means for storing the washing solution by replacing one means with the other means before using the latter. On the outlet side, a means for storing a blood flow and a washing solution flow through the mononuclear cell preparation device is provided. Or, the blood and the washing solution may optionally be separately recovered and separately treated before they are combined together.

In the case where priming is performed, a means for storing a priming solution and a means for storing a priming solution flow through the mononuclear cell preparation device are typically provided on the inlet side and the outlet side of the mononuclear cell preparation device, respectively. However, which side they are provided on depends on the flow direction of the priming solution. For these means, other lines may be provided in addition to the line(s) for the means for storing blood and the means for storing the washing solution. Or, these means may replace the means for blood and the washing solution before their own use.

Fig. 1 shows an exemplary device including all the means described above and separate lines respectively connected to these means. This mononuclear cell preparation device is designed such that a priming solution is flowed from the outlet side to the inlet side of the device. These means can be opened or closed via, for example, a stopcock appropriately in each step of the mononuclear cell preparation method of the present invention.

For reasons of workability, the means for storing blood and the means for storing a solution flow through the mononuclear cell preparation device are preferably, but not limited to, bag-shaped. Also, for reasons of workability, it is preferred that the storing means each be connected via, for example, a tube provided with a clamp, a two-way stopcock, a three-way stopcock, a roller clamp, or other means for controlling the flow and the flow rate of the corresponding solution.

The mononuclear cell recovery ratio can be calculated by dividing the mononuclear cell recovery by the granulocyte recovery. A higher ratio corresponds to higher ability to remove granulocytes. The use of a washing solution containing a divalent cation according to the present invention improves the adhesion of granulocytes to the nonwoven fabric, and therefore enables efficient preparation of mononuclear cells while reducing granulocyte contamination. The ratio of the mononuclear cell recovery to the granulocyte recovery is preferably not less than 3.5. The ratio is more preferably not less than 4.1, and still more preferably not less than 4.5.

### EXAMPLES

The following description is offered to illustrate the present invention in more detail with reference to examples below, but the present invention is not limited only to these examples.

### (Example 1)

A polycarbonate container (height (inner dimension): 7 mm, diameter (inner diameter) : 18 mm) was packed with a laminate of 40 sheets of a nonwoven fabric made of nylon 6 (air permeability: 40 mL/cm²/sec, thickness: 0.30 mm, fiber diameter: 16.0 µm) (total thickness of nonwoven fabric sheets: 12 mm). Thus, a mononuclear cell preparation device was prepared. Next, 45 mL of physiological saline (a priming solution) was charged into a 50-mL syringe, and the syringe was connected to the inlet side of the mononuclear cell preparation device through a female lock connector. All the physiological saline (45 mL) was passed through the device by pressing the plunger of the syringe slowly. Then, 5 mL of swine bone marrow, which was previously anticoagulated with 50 IU/mL heparin sodium and passed through a 70-µm cell strainer to remove coagulates, was charged into a 20-mL syringe, and the syringe was connected to the inlet side of the mononuclear cell preparation device through a porelon tube, and attached to a syringe pump. The swine bone marrow (5 mL) was applied into the mononuclear cell preparation device at a flow rate of 0.625 mL/min over 8 minutes, and a flow-through from the outlet side of the mononuclear cell preparation device was collected in a recovery container. Finally, 10 mL of physiological saline (a washing solution) was charged into a 20-mL syringe, and the syringe was connected to the inlet side of the mononuclear cell preparation device through a porelon tube. The washing solution was applied into the mononuclear cell preparation device at a flow rate of 0.625 mL/min over 16 minutes using a syringe pump, and a flow-through from the outlet side of the mononuclear cell preparation device was collected in the recovery container.

The collected flow-through (15 mL) from the mononuclear cell preparation device and the blood before the treatment were analyzed for white blood cell concentration with a blood cell counter (K-4500, available from Sysmex Corp.). Additionally, the blood before the treatment and the flow-through from the mononuclear cell preparation device were hemolyzed with FACS PharmLyse and then measured for the ratio of mononuclear cells to granulocytes with a flow cytometer (FACSCanto, available from BD). The total number of mononuclear cells was calculated by the following equation based on the white blood cell concentration and the ratio of mononuclear cells to granulocytes.
(Total number of mononuclear cells in 5 mL of blood before treatment) = (white blood cell concentration in blood before treatment) × (ratio of mononuclear cells to white blood cells in blood before treatment)×5 [mL]
(Total number of mononuclear cells in 15 mL of flow-through from mononuclear cell preparation device) = (white blood cell concentration in flow-through from mononuclear cell preparation device)×(ratio of mononuclear cells to white blood cells in flow-through from mononuclear cell preparation device)×15 [mL]

The mononuclear cell recovery [%] was calculated from the total numbers of mononuclear cells before and after the treatment as follows: (number of mononuclear cells in flow-through from mononuclear cell preparation device)/(number of mononuclear cells in blood before treatment)×100. The mononuclear cell recovery was found to be 74%.

The total number of granulocytes in the flow-through from the mononuclear cell preparation device was likewise calculated, and the granulocyte recovery [%] was also calculated as follows: (number of granulocytes in flow-through from mononuclear cell preparation device)/(number of granulocytes in blood before treatment)×100. The granulocyte recovery was found to be 49%.

The mononuclear cell recovery ratio was also calculated from the mononuclear cell recovery and the granulocyte recovery as follows: (mononuclear cell recovery)/(granulocyte recovery). The mononuclear cell recovery ratio was found to be 1.5. Table 1 shows the results.

### (Example 2)

The same container as that used in Example 1 was packed with a laminate of 24 sheets of a nonwoven fabric made of nylon 6 (air permeability: 20 mL/cm²/sec, thickness: 0.46 mm, fiber diameter: 5.0 µm) (total thickness of nonwoven fabric sheets: 11 mm), and 45 mL of physiological saline was first passed through the device from the inlet side by pressing a syringe by hand. Next, 5 mL of fresh swine bone marrow anticoagulated with heparin was passed through the device at a flow rate of 0.625 mL/min. Then, 10 mL of physiological saline was applied from the inlet side of the mononuclear cell preparation device at a flow rate of 0.625 mL. The obtained flow-through (15 mL) from the mononuclear cell preparation device was measured to calculate the mononuclear cell recovery, granulocyte recovery, and mononuclear cell recovery ratio in the same manner as in Example 1, and the results are shown in Table 1.

### (Example 3)

The same container as that used in Example 1 was packed with a laminate of 16 sheets of a nonwoven fabric made of nylon 6 (air permeability: 20 mL/cm²/sec, thickness: 0.46 mm, fiber diameter: 5.0 µm) (total thickness of nonwoven fabric sheets: 7.4 mm), and 45 mL of physiological saline was first passed through the device from the inlet side by pressing a syringe by hand. Next, 5 mL of fresh swine bone marrow anticoagulated with heparin was passed through the device at a flow rate of 0.625 mL/min. Then, 10 mL of physiological saline was applied from the inlet side of the mononuclear cell preparation device at a flow rate of 0.625 mL. The obtained flow-through (15 mL) from the mononuclear cell preparation device was measured to calculate the mononuclear cell recovery, granulocyte recovery, and mononuclear cell recovery ratio in the same manner as in Example 1, and the results are shown in Table 1.

### (Example 4)

The same container as that used in Example 1 was packed with a laminate of 16 sheets of a nonwoven fabric made of nylon 6 (air permeability: 20 mL/cm²/sec, thickness: 0.46 mm, fiber diameter: 5.0 µm) (total thickness of nonwoven fabric sheets: 7.4 mm), and 45 mL of physiological saline was first passed through the device from the inlet side by pressing a syringe by hand. Next, 5 mL of fresh swine bone marrow anticoagulated with heparin was passed through the device at a flow rate of 0.625 mL/min. Then, 10 mL of calcium chloride-containing physiological saline (calcium concentration: 36 mEq/L) was applied from the inlet side of the mononuclear cell preparation device at a flow rate of 0.625 mL. The obtained flow-through (15 mL) from the mononuclear cell preparation device was measured to calculate the mononuclear cell recovery, granulocyte recovery, and mononuclear cell recovery ratio in the same manner as in Example 1, and the results are shown in Table 1.

### (Example 5)

The same container as that used in Example 1 was packed with a laminate of 16 sheets of a nonwoven fabric made of nylon 6 (air permeability: 20 mL/cm²/sec, thickness: 0.46 mm, fiber diameter: 5.0 µm) (total thickness of nonwoven fabric sheets: 7.4 mm), and 45 mL of physiological saline was first passed through the device from the inlet side by pressing a syringe by hand. Next, 5 mL of fresh swine bone marrow anticoagulated with heparin was passed through the device at a flow rate of 0.625 mL/min. Then, 10 mL of magnesium sulfate-containing physiological saline (magnesium concentration: 34 mEq/L) was applied from the inlet side of the mononuclear cell preparation device at a flow rate of 0.625 mL. The obtained flow-through (15 mL) from the mononuclear cell preparation device was measured to calculate the mononuclear cell recovery, granulocyte recovery, and mononuclear cell recovery ratio in the same manner as in Example 1, and the results are shown in Table 1.

### (Example 6)

The same container as that used in Example 1 was packed with a laminate of 16 sheets of a nonwoven fabric made of nylon 6 (air permeability: 20 mL/cm²/sec, thickness: 0.46 mm, fiber diameter: 5.0 µm) (total thickness of nonwoven fabric sheets: 7.4 mm), and 45 mL of physiological saline was first passed through the device from the inlet side by pressing a syringe by hand. Next, magnesium sulfate was added to 5 mL of fresh swine bone marrow anticoagulated with heparin to a final magnesium concentration of 34 mEq/L, and the resulting bone marrow was passed through the device at a flow rate of 0.625 mL/min. Then, 10 mL of magnesium sulfate-containing physiological saline (magnesium concentration: 34 mEq/L) was applied from the inlet side of the mononuclear cell preparation device at a flow rate of 0.625 mL. The obtained flow-through (15 mL) from the mononuclear cell preparation device was measured to calculate the mononuclear cell recovery, granulocyte recovery, and mononuclear cell recovery ratio in the same manner as in Example 1, and the results are shown in Table 1.

### (Example 7)

The same container as that used in Example 1 was packed with a laminate of 40 sheets of a nonwoven fabric made of nylon 6 (air permeability: 65 mL/cm²/sec, thickness: 0.19 mm, fiber diameter: 2.7 µm) (total thickness of nonwoven fabric sheets: 7.6 mm), and 45 mL of physiological saline was first passed through the device from the inlet side by pressing a syringe by hand. Next, 5 mL of fresh swine bone marrow anticoagulated with heparin was passed through the device at a flow rate of 0. 625 mL/min. Then, 10 mL of physiological saline was applied from the inlet side of the mononuclear cell preparation device at a flow rate of 0.625 mL. The obtained flow-through (15 mL) from the mononuclear cell preparation device was measured to calculate the mononuclear cell recovery, granulocyte recovery, and mononuclear cell recovery ratio in the same manner as in Example 1, and the results are shown in Table 1.

### (Comparative Example 1)

The same container as that used in Example 1 was packed with a laminate of 28 sheets of a nonwoven fabric made of polypropylene (air permeability: 33 mL/cm²/sec, thickness: 0.43 mm, fiber diameter: 5.7 µm) (total thickness of nonwoven fabric sheets: 12 mm), and 45 mL of physiological saline was first passed through the device from the inlet side by pressing a syringe by hand. Next, 5 mL of fresh swine bone marrow anticoagulated with heparin was passed through the device at a flow rate of 0. 625 mL/min. Then, 10 mL of physiological saline was applied from the inlet side of the mononuclear cell preparation device at a flow rate of 0.625 mL. The obtained flow-through (15 mL) from the mononuclear cell preparation device was measured to calculate the mononuclear cell recovery, granulocyte recovery, and mononuclear cell recovery ratio in the same manner as in Example 1, and the results are shown in Table 1.

### (Comparative Example 2)

The same container as that used in Example 1 was packed with a laminate of 20 sheets of a nonwoven fabric made of polybutylene terephthalate (air permeability: 36 mL/cm²/sec, thickness: 0.61 mm, fiber diameter: 4.6 µm) (total thickness of nonwoven fabric sheets: 12 mm), and 45 mL of physiological saline was first passed through the device from the inlet side by pressing a syringe by hand. Next, 5 mL of fresh swine bone marrow anticoagulated with heparin was passed through the device at a flow rate of 0.625 mL/min. Then, 10 mL of physiological saline was applied from the inlet side of the mononuclear cell preparation device at a flow rate of 0.625 mL. The obtained flow-through (15 mL) from the mononuclear cell preparation device was measured to calculate the mononuclear cell recovery, granulocyte recovery, and mononuclear cell recovery ratio in the same manner as in Example 1, and the results are shown in Table 1.

### (Comparative Example 3) Preparation of mononuclear cells by Ficoll-Paque fractionation

An amount of 2 mL of fresh swine bone marrow anticoagulated with heparin was combined and diluted with 2 mL of physiologic saline. Next, 3 mL of Ficoll Paque-Plus solution (GE Healthcare Japan) was added to a 15-mL centrifuge tube, and the bone marrow diluted as described above was layered on the Ficoll solution. The tube was centrifuged in a centrifuge at 1400 rpm for 30 minutes to recover a mononuclear cell fraction. In order to remove the Ficoll solution, the recovered mononuclear cell fraction was combined with 10 mL of physiologic saline, and the mixture was centrifuged in the centrifuge at 1500 rpm for 10 minutes. Then, the supernatant was removed, and the remaining fraction was again combined with 10 mL of physiologic saline, and then the mixture was centrifuged at 1500 rpm for 10 minutes. The supernatant was again removed, and the remaining fraction was combined with physiological saline to a total fluid volume of 1 mL. The mononuclear cell recovery, granulocyte recovery, and mononuclear cell recovery ratio of the resulting solution were calculated in the same manner as in Example 1, and the results are shown in Table 1.

Although the Ficoll-Paque fractionation achieved almost the same mononuclear cell recovery and granulocyte recovery as those of Example 2, it had problems such as time-consuming processes such as time-consuming centrifugation, complicated operations, high inter-operator variability, and the need of operations in an open system.

**[Table 1]**

| | Material | *Addition of magnesium sulfate to bone marrow | Washing solution | Air permeability | Nonwoven fabric Total thickness | Fiber diameter | Mononuclear cell recovery | Granulocyte recovery | Mononuclear cell recovery ratio |
|---|---|---|---|---|---|---|---|---|---|
| | | | | [mL/cm²/sec] | [mm] | [µm] | [%] | [%] | |
| Example 1 | Nylon | (-) | Physiological saline | 40 | 12 | 16 | 74 | 49 | 1.5 |
| Example 2 | Nylon | (-) | Physiological saline | 20 | 11 | 5 | 39 | 3 | 13 |
| Example 3 | Nylon | (-) | Physiological saline | 20 | 7.4 | 5 | 46 | 13 | 3.5 |
| Example 4 | Nylon | (-) | Calcium chloride-containing physiological saline | 20 | 7.4 | 5 | 53 | 8 | 6.4 |
| Example 5 | Nylon | (-) | Magnesium sulfate-containing physiological saline | 20 | 7.4 | 5 | 43 | 8 | 5.4 |
| Example 6 | Nylon | (+) | Magnesium sulfate-containing physiological saline | 20 | 7.4 | 5 | 50 | 40 | 1.3 |
| Example 7 | Nylon | (-) | Physiological saline | 65 | 7.6 | 2.7 | 33 | 8 | 4.1 |
| Comparative Example 1 | PP | (-) | Physiological saline | 33 | 12 | 5.7 | 81 | 84 | 1 |
| Comparative Example 2 | PBT | (-) | Physiological saline | 36 | 12 | 4.6 | 74 | 82 | 0.9 |
| Comparative Example 3 | - | - | - | - | - | - | 41 | 5 | 8.2 |

| | | | | | | | | | |
|---|---|---|---|---|---|---|---|---|---|
| * Final magnesium concentration: 34 mEq/L | | | | | | | | | |

### REFERENCE SIGNS LIST

- 1: Chamber
- 2: Container packed with mononuclear cell preparation material (mononuclear cell preparation device)
- 3: Means for storing priming solution flow through mononuclear cell preparation device
- 4: Means for storing blood
- 5: Means for storing washing solution
- 6: Means for storing priming solution
- 7: Means for storing blood flow and washing solution flow through mononuclear cell preparation device
- 8 to 10: Three-way stopcock
- 11 to 19: Line

## Claims

1. A mononuclear cell preparation method, comprising the steps of:
(A) passing blood through a mononuclear cell preparation device from an inlet side to an outlet side of the device, thereby capturing granulocytes in the mononuclear cell preparation device; and
(B) passing a washing solution comprising a divalent cation through the mononuclear cell preparation device from the inlet side to the outlet side of the device, thereby recovering mononuclear cells remaining in the mononuclear cell preparation device,
wherein the mononuclear cell preparation device comprises a container provided with an inlet and an outlet and is packed with a mononuclear cell preparation material for removing granulocytes from blood to prepare mononuclear cells, wherein the mononuclear cell preparation material comprises a nonwoven fabric made of a polyamide resin and having an air permeability of at least 10 mL/cm²/sec but not more than 75 mL/cm²/sec.

2. The mononuclear cell preparation method according to claim 1, comprising, before the step (A), the step of priming.

3. The mononuclear cell preparation method according to claim 1 or 2,
wherein the divalent cation is calcium ion or magnesium ion.

4. The mononuclear cell preparation method according to any one of claims 1 to 3,
wherein the divalent cation is present at a concentration of not more than 500 mEq/L.

5. The mononuclear cell preparation method according to any one of claims 1 to 4,
wherein a mononuclear cell preparation flow-through from the outlet side of the mononuclear cell preparation device has a ratio of a mononuclear cell recovery to a granulocyte recovery of not less than 3.5.

## Patentansprüche

1. Mononukleäres Zellpräparationsverfahren, umfassend die folgenden Schritte:
(A) Passieren von Blut durch eine mononukleäre Zellpräparationsvorrichtung von einer Einlassseite zu einer Auslassseite der Vorrichtung, wodurch Granulozyten in der mononukleären Zellpräparationsvorrichtung eingefangen werden; und
(B) Passieren einer Waschlösung, umfassend ein zweiwertiges Kation, durch die mononukleäre Zellpräparationsvorrichtung von der Einlassseite zu der Auslassseite der Vorrichtung, wodurch in der mononukleären Zellpräparationsvorrichtung verbliebene mononukleäre Zellen rückgewonnen werden,
wobei die mononukleäre Zellpräparationsvorrichtung einen mit einem Einlass und einem Auslass ausgestatteten Behälter umfasst und mit einem mononukleären Zellpräparationsmaterial zur Entfernung der Granulozyten aus dem Blut zur Herstellung mononukleärer Zellen gepackt ist, wobei das mononukleäre Zellpräparationsmaterial einen Vliesstoff umfasst, das aus einem Polyamidharz hergestellt ist und eine Luftpermeabilität von zumindest 10 ml/cm²/sek., aber nicht mehr als 75 ml/cm²/sek aufweist.

2. Mononukleäres Zellpräparationsverfahren nach Anspruch 1, umfassend, vor dem Schritt (A), den Schritt des Primings.

3. Mononukleäres Zellpräparationsverfahren nach Anspruch 1 oder 2, wobei das zweiwertige Kation ein Calciumion oder ein Magnesiumion ist.

4. Mononukleäres Zellpräparationsverfahren nach einem der Ansprüche 1-3, wobei das zweiwertige Kation mit einer Konzentration von nicht mehr als 500 mEq/l vorliegt.

5. Mononukleäres Zellpräparationsverfahren nach einem der Ansprüche 1-4, wobei der mononukleäre Zellpräparationsdurchfluss von der Auslassseite der mononukleären Zellpräparationsvorrichtung ein Verhältnis einer mononukleären Zellrückgewinnung zu einer Granulozyten-Rückgewinnung von nicht weniger als 3,5 aufweist.

## Revendications

1. Procédé de préparation de cellules mononucléaires, comprenant les étapes suivantes :
(A) le passage de sang à travers un dispositif de préparation de cellules mononucléaires d'un côté d'entrée à un côté de sortie du dispositif pour ainsi capturer des granulocytes dans le dispositif de préparation de cellules mononucléaires ; et
(B) le passage d'une solution de lavage comprenant un cation divalent à travers le dispositif de préparation de cellules mononucléaires du côté d'entrée au côté de sortie du dispositif pour ainsi récupérer les cellules mononucléaires restant dans le dispositif de préparation de cellules mononucléaires,
dans lequel le dispositif de préparation de cellules mononucléaires comprend un récipient qui est pourvu d'une entrée et d'une sortie et qui est rempli d'un matériau de préparation de cellules mononucléaires permettant de retirer les granulocytes du sang pour préparer des cellules mononucléaires,
dans lequel le matériau de préparation de cellules mononucléaires comprend un tissu non tissé formé d'une résine de polyamide et présentant une perméabilité à l'air d'au moins 10 ml/cm²/s, mais qui n'est pas supérieure à 75 ml/cm²/s.

2. Procédé de préparation de cellules mononucléaires selon la revendication 1, comprenant, avant l'étape (A), l'étape d'amorçage.

3. Procédé de préparation de cellules mononucléaires selon la revendication 1 ou 2,
dans lequel le cation divalent est un ion calcium ou un ion magnésium.

4. Procédé de préparation de cellules mononucléaires selon l'une quelconque des revendications 1 à 3,
dans lequel le cation divalent est présent en concentration de pas plus 500 mEq/L.

5. Procédé de préparation de cellules mononucléaires selon l'une quelconque des revendications 1 à 4,
dans lequel un écoulement traversant de préparation de cellules mononucléaires à partir du côté de sortie du dispositif de préparation de cellules mononucléaires présente un rapport de la récupération de cellules mononucléaires à la récupération de granulocytes qui n'est pas inférieur à 3,5.
